(19) Europäisches Patentamt
European Patent Office
Office européen des brevets

(11) Publication number: 0 268 396 B1

(12) **EUROPEAN PATENT SPECIFICATION**

(45) Date of publication of the patent specification: **28.11.90**

(51) Int. Cl.⁵: **C07D 333/34,** C07D 307/64, C07D 409/12, A61K 31/38, A61K 31/34

(21) Application number: **87309465.0**

(22) Date of filing: **27.10.87**

(54) **4-(benzoyl)thiophene(or furan)-sulfonamide and derivatives thereof for the topical treatment of elevated intraocular pressures.**

(30) Priority: **03.11.86 US 926200**

(43) Date of publication of application: **25.05.88 Bulletin 88/21**

(45) Publication of the grant of the patent: **28.11.90 Bulletin 90/48**

(84) Designated Contracting States: **CH DE FR GB IT LI NL**

(56) References cited:
**GB-A- 1 468 111**
**US-A- 4 486 444**

**Report of the 8th Intern. Congress of the Eye Research, Sept. 4-8, 1988: Carbonic Anhydrase Inhibitors (CA/s):Correlation between Reactivity with reduced Glutathione (GHS) and Induction of Contact Sensitivity in the Guinea Pig. (H Schwam, S R Michelson, S J de Solms, P Duprat, P Gautheron, K L Shepard, R L Smith, M F Sugrue)B. 1985), 480-4 000**

**The file contains technical information submitted after the application was filed and not included in this specification**

(73) Proprietor: **MERCK & CO. INC., 126, East Lincoln Avenue P.O. Box 2000, Rahway New Jersey 07065-0900(US)**

(72) Inventor: **Hartman, George D., 1529 Tennis Circle, Lansdale Pennsylvania 19446(US)**
Inventor: **Halczenko, Wasyl, 1003 Clymer Road, Hatfield Pennsylvania 19440(US)**

(74) Representative: **Hesketh, Alan, Dr., European Patent Department Merck & Co., Inc. Terlings Park Eastwick Road, Harlow Essex, CM20 2QR(GB)**

EP 0 268 396 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

SUMMARY OF THE INVENTION

This invention relates to 4-(benzoyl)thiopene (or furan)-2-sulfonamide and novel derivatives thereof which are useful in the treatment of elevated intraocular pressure. More particularly this invention relates to compounds having the structural formula:

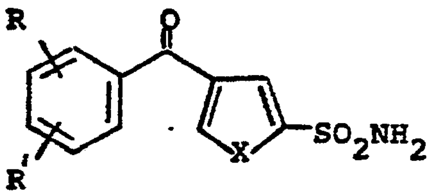

wherein -X- is -O- or -S- and
R and $R^1$ are as hereinafter defined, and the ophthalmologically acceptable salts thereof. This invention also relates to ophthalmic compositions that are employed in the treatment of elevated intraocular pressure, especially when accompanied by pathological damage such as in the disease known as glaucoma.

BACKGROUND OF THE INVENTION

Glaucoma is an ocular disorder associated with elevated intraocular pressures which are too high for normal function and may result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Indeed, few advances were made in the treatment of glaucoma since pilocarpine and physostigmine were introduced.

Some β-adrenergic blocking agents are effective in reducing intraocular pressure, but while many of these agents are effective, they also have other characteristics, e.g. membrane stabilizing (local anesthetic) activity, that make them unacceptable for chronic ocular use.

(S)-1- tert-butylamino-3-[(4-morpholino-1,2,5-thiadiazol-3-yl)oxy]- -propanol, a β-adrenergic blocking agent, was found to reduce intraocular pressure and to be devoid of many unwanted side effects associated with pilocarpine and, in addition, to possess advantages over many other β-adrenergic blocking agents, e.g., to be devoid of local anesthetic properties, to have a long duration of activity, and to display minimal tolerance.

Although pilocarpine, physostigmine and β-block agents reduce intraocular pressure, none of these drugs manifests its action by inhibiting the enzyme carbonic anhydrase and, thereby, impeding the contribution made by the carbonic anhydrase pathway to aqueous humor formation.

Agents referred to as carbonic anhydrase inhibitors, block or impede this inflow pathway by inhibiting the enzyme, carbonic anhydrase. While such carbonic anhydrase inhibitors are now used to treat elevated intraocular pressure by oral, intravenous or other systemic routes, they thereby have the distinct disadvantage of inhibiting carbonic anhydrase throughout the entire body. Such a gross disruption of a basic enzyme system is justified only during an acute attack of alarmingly elevated intraocular pressure, or when no other agent is effective. Despite the desirability of directing the carbonic anhydrase inhibitor only to the desired ophthalmic target tissue, no topically effective carbonic anhydrase inhibitors are available for clinical use.

However, topically effective carbonic anhydrase inhibitors are reported in E.P. Publication 129,478; and U.S. Patents 4,416,890; 4,486,444; and 4,542,152. The latter two patents deal with subject matter closely related to the present invention; 5-phenylsulfonylthiophene-2-sulfonamides and 5-benzoylthiophene-2-sulfonamides, respectively.

To be an effective and acceptable topical agent, an ophthalmic carbonic anhydrase inhibitor must not only penetrate the ophthalmic tissues to reach the active sites within the eye, but it must also be devoid of those side effects including irritation, allergic reaction and the like which would preclude long term administration.

DETAILED DESCRIPTION OF THE INVENTION

The novel compounds of the present invention are represented by structural formula I:

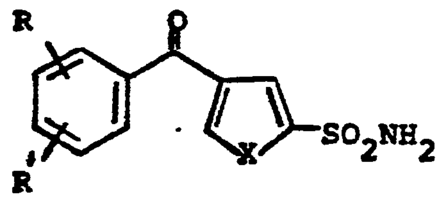

or an ophthalmologically acceptable salt thereof,
wherein:
X is S or O; and
R and $R^1$ are independently
1) hydrogen;
2) hydroxy;
3) halo such as fluoro or chloro;
4) nitro;
5) perfluoro-$C_{1-3}$alkyl;
6) $C_{1-6}$alkyl;
7) $C_{1-6}$alkoxy;
8) -$(C_nH_{2n})$-$NR^2R^3$, wherein n is 0–3 and $R^2$ and $R^3$ are independently
a) hydrogen;
b) $C_{1-8}$alkyl;
c) monocyclic aryl-$C_{1-3}$alkyl, wherein the aryl group is phenyl, pyridyl, imidazolyl, furyl, thienyl or the like;
d) hydroxy-$C_{2-6}$alkyl
e) $C_{1-3}$alkoxy-$C_{2-6}$alkyl;
f) $R^4R^5N$-$C_{2-4}$alkyl; wherein $R^4$ and $R^5$ are independently hydrogen or $C_{1-3}$alkyl;
g) $R^2$ and $R^3$ if lower alkyl taken together either directly or through a second hetero atom selected from 0, S or $NR^{20}$ form 5- to 7-membered heterocycle with the nitrogen atom to which they are attached, such as piperidyl, pyrrolyl, azepinyl, piperazinyl, 4-methylpiper-azinyl, 4-benzylpiperazinyl, morpholinyl, thiomorpholinyl or the like, $R^{20}$ representing hydrogen, $C_{1-8}$alkyl, monocyclic aryl-$C_{1-3}$alkyl, hydroxy-$C_{2-6}$alkyl, $C_{1-3}$alkoxy-$C_{2-6}$alkyl, or $R^4R^5N$-$C_{2-4}$alkyl in wich $R^4$ and $R^5$ are as defined above;
h)

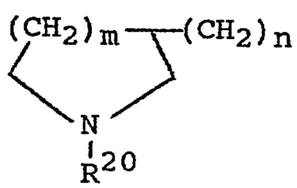

wherein m is 1 or 2, and $R^{20}$ is as defined above.

In the foregoing definitions and in what follows in this specification, the term "alkyl" and "alkoxy" refer to groups which, if comprised of more than two carbon atoms, the alkyl moiety is stright chain, branched chain or cyclic.

In a preferred embodiment of the novel compounds X is -S-.

It is more preferred that X is -S- and one of R and $R^1$ is hydrogen or hydroxy and the other is $C_{1-6}$alkyl, $C_{1-6}$alkoxy, or -$(C_nH_{2n})$-$NR^2R^3$, especially wherein n is 1 and $R^2$ and $R^3$ are both $C_{1-8}$alkyl.

The novel process for preparing compounds wherein R and/or $R^1$ is -OH comprises heating the corresponding methoxy compound with 48% HBr at or near the reflux temperature for about 1 to 5 hours.

A process for preparing compounds in which the phenyl, moiety has an aminoalkyl substituent comprises treating the appropriate amine with the corresponding bromoalkylphenyl compound at about 15 to 30° C for about 1 to 5 days in an ethereal solvent such as THF, diethyl ether, 1,2-dimethoxyethane or the like.

In some instances it is convenient to form the sulfonamide group as a last step. This process involves treating the corresponding sulfonyl chloride in an inert organic solvent such as a chlorinated hydrocarbon, e.g., chloroform, dichloromethane or the like with excess gaseous ammonia at about -10 to 30°C followed by aging the suspension of product for about 1 to 5 hours to assure completion of the reaction.

This invention is also concerned with formulations adapted for topical ocular administration for the treatment of glaucoma and other stages of elevated intraocular pressure which contain about 0.1% to 15% by weight of medicament, especially about 0.5 to 2% by weight of medicament, the remainder being comprised of carriers and other excipients well known in the art.

The medicament in the novel topical ocular formulations comprises one of the novel compounds of this invention either alone or in combination with a β-adrenergic blocking agent such as timolol maleate or a

parasympathomimetic agent such as pilocarpine. In such combinations the two active agents are present in approximately equal amounts.

The novel method of treatment of this invention comprises the treatment of elevated intraocular pressure and glaucoma by the administration of the novel compound of this invention or a pharmaceutical formulation thereof. Of primary concern is the treatment by topical ocular administration of about 0.1 to 25 mg and especially 0.2 to 10 mg of such compound per day, either by single dose or on a 2 to 4 does per day regimen.

The utility of the novel compounds was determined from the observation that the intraocular pressure (IOP) of the α-chymotrypsinized rabbit eye was significantly lowered by the bilateral instillation of solutions or suspensions of a representative number of the compounds shown in the following table:

**EFFECT OF TOPICALLY ADMINISTERED DRUG ON THE α-CHYMOTRYPSIN-INDUCED ELEVATION OF IOP IN THE RABBIT (a)**

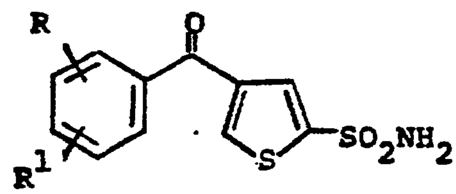

| TEST COMPOUND | | DOSE (%)[b] | MAX. IOP DROP (mm Hg)[c] |
|---|---|---|---|
| R | R1 | | |
| H | 4-*n*-BuNHCH$_2$- | 0.5 | -3.0 |
| H | 3-*i*-BuNHCH$_2$- | 0.5 | -7.3 |
| H | 3-*i*-BuN(CH$_3$)CH$_2$- | 0.5 | -4.0 |
| H | 3-( 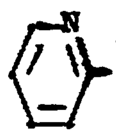 CH$_2$NHCH$_2$-) | 0.5 | -4.0 |

a) Rabbits were pretreated with α-chymotrypsin at least 1 month previously in right eye only. Compound or vehicle (0.5% HEC) was instilled (50 µl) into both eyes. For full protocol see Sugrue et al, J. Pharmacol. Exp. Ther., 232, 534 (1985).

b) A single 50 µl drop of the test compound was applied topically as a formulation of the indicated % concentration in freshly prepared hydroxyethylcellulose (HEC) vehicle.

c) The reported number is the maximum, statistically significant drop in IOP recorded during the 5 hour duration of the assay.

EXAMPLE 1

4-(4-methylbenzoyl)thiophene-2-sulfonamide

Step A: Preparation of 3-(4-Methylbenzoyl)thiophene

To 50.0 g (0.307 mol) 3-bromothiophene in 300 ml of ether cooled to -75°C under $N_2$ was added 0.307 moles of n-butyllithium (in hexane) dropwise. After addition the reaction mixture was stirred at -75° for 1 hour and then treated with a solution of 36.3 g (0.310 mole) 4-methylbenzonitrile in 65 ml ether dropwise at <-70°C. The resulting mixture was stirred at -75°C for 1 hour and then allowed to gradually warm to -10°C over 2 hours. The reaction mixture was quenched with 20 ml $H_2O$, followed by 2N HCl until all the solid dissolved. The aqueous solution was heated at reflux for 2.5 hours, cooled and extracted with ether. The ether extract was dried and stripped to give the product as an oil.

Step B: Preparation of 4-(4-Methylbenzoyl)-thiophene-2-sulfonic acid

To 3.0 g (0.0148 mol) of product from Step A in 25 ml $CH_2Cl_2$ cooled to 0-10°C was added 4.8 g (0.047 mol) acetic anhydride followed by 1.57 g (0.016 mol) concentrated sulfuric acid. After stirring for 48 hours at room temperature, the reaction mixture was cooled, 40 ml of hexane was added, and the crude desired product was collected as a tan solid.

Step C: Preparation of 4-(4-Methylbenzoyl) thiophene-2-sulfonyl chloride

To 30.7 g (0.109 mol) of product from Step B and 2.19 g (0.03 mol) dimethylformamide in 250 ml THF at 0-10°C was added dropwise 41.15 g (0.327 m) oxalyl chloride. After stirring for 16 hours at room temperature the solvent was removed invacuo and the residue was treated with 50 ml $H_2O$ and 300 ml $CH_2Cl_2$. The organic layer was dried and concentrated to dryness to provide the sulfonyl chloride as a solid, m.p. 117-119°C.

Step D: Preparation of 4-(Methylbenzoyl)thiophene-2-sulfonamide

To 1.0 g (0.0033 mol) of product from Step C in 35 ml $CHCl_3$ was added excess gaseous $NH_3$ and the resulting suspension was stirred for 3 hours. The reaction mixture was then concentrated to dryness and the residue was purified by flash chromatography on silica gel eluting with 5% $CH_3OH/CHCl_3$ to provide pure product, m.p. 175-177°C.

EXAMPLE 2

4-(4-Methoxybenzoyl)thiophene-2-sulfonamide

Step A: Preparation of 3-(4-Methoxybenzoyl)thiophene

To 4.0 g (0.0246 m) of 3-bromothiophene in 70 ml ether cooled to -75°C under $N_2$ was added dropwise 0.0246 m of n-butyllithium (in hexane) at such a rate that the temperature was <-70°C. After addition was complete the reaction mixture was stirred at -75°C for 10 minutes and then a solution of 4-methoxybenzonitrile (3.46 g, 0.026 m) in 20 ml ether was added dropwise at <-70°C. The resulting suspension was stirred at -75°C for 45 minutes and then allowed to warm to -10°C over 2 hours. The reaction mixture was cooled, quenched with 15 ml $H_2O$ and then with 40 ml of 2N HCl. The p e separated and the aqueous extract was washed with 50 ml ether, and then heated at reflux for 2 hours. This solution was cooled and extracted with 2 x 60 ml ether. The organic phases were washed with brine, dried and evaporated to give an oil which was triturated with petroleum ether to give the product as a solid, m.p. 58-61°C.

Step B: Preparation of 4-(4-Methoxybenzoyl)thiophene-2-sulfonic acid

9.7 g (0.05 mole) of product from Step A was dissolved in 75 ml $CH_2Cl_2$ and cooled to -10°C. Then, 15.3 g (0.15 mole) acetic anhydride was added dropwise followed by the dropwise addition of 5.9 g (0.06 mol) concentrated sulfuric acid. The resulting mixture was stirred at room temperature overnight.

The yellow solid was collected on a filter, washed with $CH_2Cl_2$ and air dried to provide the desired product.

Step C: Preparation of 4-(4-Methoxybenzoyl)thiophene-2-sulfonamide

To 2.98 g (0.01 mol) of product from Step B suspended in 75 ml $CH_2Cl_2$ was added excess thionyl chloride and the resulting suspension was stirred, and heated at reflux for 1.5 hours to give a homogeneous solution. The thionyl chloride was evaporated and the residue was extracted with $CHCl_3$ after ice had been added. The organic extract was washed with brine, dried and the solvent evaporated to give a yellow oil. This was taken up in acetone and treated with 5 ml of concentrated $NH_4OH$ dropwise. After stirring for 15 minutes, this was extracted with chloroform and the extract was dried. Filtration and evaporation of the solvent gave the desired product as a yellow solid, m.p. 173-175°C.

EXAMPLE 3

4-(4-Hydroxybenzoyl)thiophene-2-sulfonamide

The product from Example 2 (0.95 g, 0.0032 mole) was suspended in 15 ml 48% aqueous HBr and heated at reflux for 2.5 hours. The reaction mixture was cooled, poured into ice water and extracted with 4 x 20 ml portions of ethyl acetate. The combined organic extracts were dried and evaporated to give the title compound, m.p. 192-193°C.

EXAMPLE 4

4-(4-Methylaminomethylbenzoyl)thiophene-2-sulfonamide

Step A: Preparation of 4-(4-Bromomethylbenzoyl)-thiophene-2-sulfonyl chloride

To 5.0 g (0.0167 mol) 4-(4-methylbenzoyl)-thiophene-2-sulfonyl chloride in 60 ml $CHCl_3$ was added 5.9 g (0.033 mol) of N-bromosuccinimide and 100 mg of benzoyl peroxide. The resulting solution was refluxed and simultaneously irradiated with a sun lamp. After 0.5 hours the reaction mixture was cooled and extracted with 2 x 100 ml $H_2O$. The organic phase was washed with 50 ml 5% sodium thiosulfate, brine and was dried. Solvent evaporation left the title compound as a reddish oil.

Step B: Preparation of 4-(4-Bromomethylbenzoyl)-thiophene-2-sulfonamide

Into a solution of 5.0 g (0.013 mol) of product from Step A in 50 ml $CHCl_3$ was bubbled $NH_3$ (gas) at room temperature. The resulting suspension was stirred at room temperature until all the starting material was gone (~2.5 hours) as shown by tlc. The solvent was then removed invacuo and the residue was purified by flash chromatography on silica gel eluting with 5% methanol/chloroform to provide pure 4-(4-bromomethylbenzoyl)thiophene-2-sulfonamide, m.p. 172-180°C.

Step C: Preparation of 4-(4-Methylaminomethyl-benzoyl)thiophene-2-sulfonamide

To a solution of 0.1 g (0.26 mmol) of product from Step B in 5 ml tetrahydrofuran at room temperature was added excess gaseous methylamine. The resulting solution was stirred at room temperature for 2 days. The solvent was then evaporated, and the residue was dissolved in $H_2O$ and extracted with 4 x 25 ml portions of ethyl acetate. The aqueous phase was then adjusted to pH 8 and extracted with ethyl acetate. The solvent was removed, the residue was taken up in $H_2O$, adjusted to pH 1 and extracted with ethyl acetate. The aqueous phase was basified with $NH_4OH$ solution and extracted with ethyl acetate. This extract was dried and evaporated to a yellow residue which was purified by flash chromatography on silica gel, eluting with $CHCl_3$ saturated with $NH_3$ (9): methanol (1) to give pure tile compound as a tan solid. This was treated with ethanol saturated with HCl to provide the HCl salt, m.p. 237-239°C (dec).

Employing the procedures substantially as described in Example 4, but substituting for the methylamine used in Step C thereof, the amines depicted in Table I, there were produced the aminoalkyl compounds also depicted in Table I:

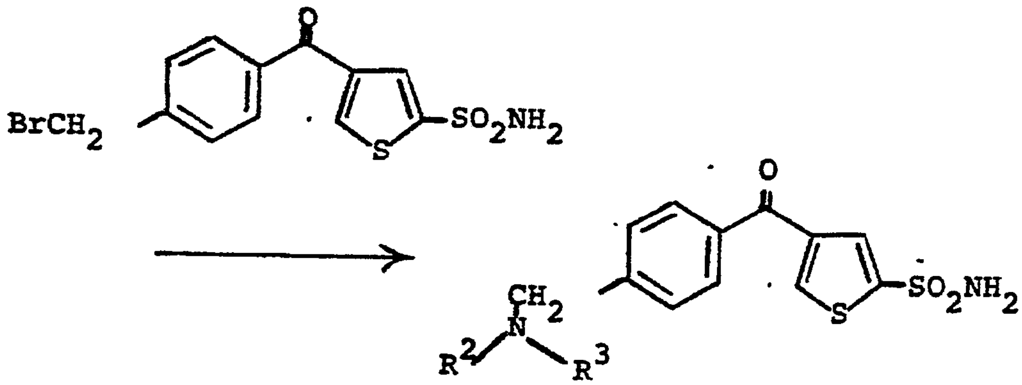

TABLE I

| $R^2R^3N-$ | m.p. (°C) |
|---|---|
| $i-C_4H_9NH-$ | 237-239° (HCl) |
| $C_6H_5CH_2NH-$ | 244-246° (HCl) |
| 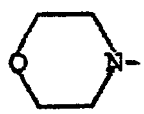 | 164-169° |
| 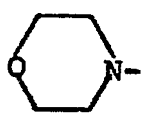 | 232-235° (HCl) |
| $n-C_4H_9NH-$ | 243-246° (HCl) |
| 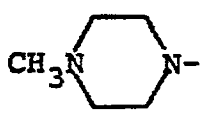 | 250-252° (2HCl) |
| 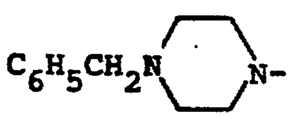 | 213-220° (2HCl) |
| 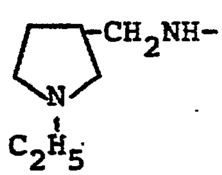 | 237-241° (2HCl) |

EXAMPLE 5

4-[3-(2-Pyridylmethylaminomethyl)]benzoylthiophene-2-sulfonamide dihydrochloride

Step A: Preparation of 3-(3-Methylbenzoyl)thiophene

To 0.307 mol n-butyllithium in 300 ml ether at -75°C under $N_2$ was added dropwise a solution of 50.0 g (0.307 mol) 3-bromothiophene in 30 ml ether at <-70°C. This was stirred 1 hour at -75°C and then a solution of 41.0 g (0.35 mol) 3-methylbenzonitrile in 25 ml ether was added dropwise at <-70°C. This was stirred at -75°C for 1.5 hours, -50°C for 1.0 hour, and then allowed to warm to -10°C over 1.5 hours.

The reaction was quenched with 30 ml $H_2O$ and enough 2 N HCl to dissolve the resulting solid. The aqueous phase was separated, refluxed for 2.5 hours, then cooled and extracted with ether. The organic extract was dried and the solvent evaporated to give the title compound as an oil.

Step B: Preparation of 4-(3-Methylbenzoyl)thiophene-2-sulfonic acid potassium salt

To 56.0 g (0.185 mol) of product from Step A suspended in 200 ml of ethyl acetate and cooled to 0-10 °C was added 22.7 g (0.232 mol) concentrated sulfuric acid followed by 37.74 g (0.37 m) acetic anhydride and the resulting solution was stirred overnight. The cooled reaction mixture was quenched with 20 ml ethanol followed by a solution of 22.0 g (0.225 mol) potassium acetate in 140 ml of 3% aqueous ethanol. The resulting solid was collected by filtration, washed with 1/1 ethanol/ethyl acetate and air dried to provide the title compound, m.p. 170-180°C.

Step C: Preparation of 4-(3-Methylbenzoyl)thiophene-2-sulfonyl chloride

To 10.0 g (0.031 mol) of product from Step B suspended in 125 ml of ethyl acetate at 0-10°C was added 7.9 g (0.0625 mol) oxalyl chloride followed by the dropwise addition of 0.7 g (0.0016 mol) dimethylformamide. The resulting suspension was stirred vigorously at room temperature overnight, then cooled and quenched with brine. The organic phase was separated, dried and evaporated. The residue was recrystallized from isopropanol to provide pure title compound, m.p. 114-116°C.

Step D: Preparation of 4-(3-Bromomethylbenzoyl) thiophene-2-sulfonyl chloride

A solution of 10.0 g (0.033 mol) of product from Step C, 16.7 g (0.094 mol) N-bromosuccinimide, and 100 mg benzoyl peroxide in 125 ml chloroform was heated at reflux and simultaneously irradiated with a sun lamp (275 watt) for 15 minutes. The reaction mixture was then cooled and extracted with water. The organic phase was separated, washed with 5% sodium thiosulfate solution, brine, then dried and evaporated to give the title compound as an oil.

Step E: Preparation of 4-(3-Bromomethylbenzoyl)-thiophene-2-sulfonamide

To 12.5 g (0.33 mol) of product from Step D dissolved in 125 ml $CHCl_3$ cooled to 0-10°C was added $NH_3$ (gas) over 5 minutes. The resulting suspension was stirred until starting material was gone (~2 hours). The solvent was then evaporated and the residue was ied by flash chromatographyon silica gel eluting with 5% methanol/chloroform to afford pure title compound as an oil ($R_f$ 0.5).

Step F: Preparation of 4-[3-(2-Pyridylmethylaminomethyl)]benzoylthiophene-2-sulfonamide dihydrochloride

To 3.60 g (0.01 mol) of product from Step E in 50 ml tetrahydrofuran was added 4.37 g (0.44 mol) 2-aminomethylpyridine and the resulting solution was stirred for 4 days at room temperature. The solvent was evaporated and the residue was made acidic, and extracted with ethyl acetate and the aqueous phase was neutralized with ammonium hydroxide. This solution was extracted with ethyl acetate and the solvent evaporated to give a residue that was purified by flash chromatography on silica gel, eluting with 10% methanol/chloroform. Solvent removal gave an oil which was treated with ethanolic HCl to provide the title compound as a white solid with m.p. 95-105°C.

In a manner analogous to Example 5 there were also prepared:
4-[3-(4-methyl-l-piperazinyl)methyl]benzoyl]thiophene-2-s lfonamide, m.p. 258-261°C.
4-[3-(Isobutylaminomethyl)benzoyl]thiophene-2-sulfonamid , m.p. 246-248°C.
4-[3-(N-Isobutyl-N-methyl)aminomethyl]benzoylthiophene-2 sulfonamide, m.p. 238-240°.
4-[3-(Morpholinomethyl)benzoyl]thiophene-2-sulfonamide, m.p. 253-255°.

EXAMPLE 6

4-[3-(N,N-Dimethylaminomethyl)-4-hydroxy]benzoylthiophene-2-sulfon mide hydrochloride

To 1.70 g (6 mmol) 4-(4-hydroxy)benzoylthiophene-2-sulfonamide in 15 ml ethanol was added 2.71 g (24 mmole) dimethylamine (40% aqueous solution) and 0.73 g (9 mmole) formaldehyde (37% aqueous solution) and the resulting solution was heated at reflux for 16 hours.

The cooled reaction mixture was filtered and the filtrate was concentrated to dryness invacuo. The resulting residue was purified by flash chromatography on silica gel eluting with chloroform (9)/methanol (1) to give a pale yellow solid. This was recrystallized from isopropanol. The resulting solid, which was the pure free base, was treated with HCl (gas) in ethanol to provide the product, m.p. 230-232°C.

Employing the procedures substantially as described above but substituting the appropriate amine component, the following compounds were made.

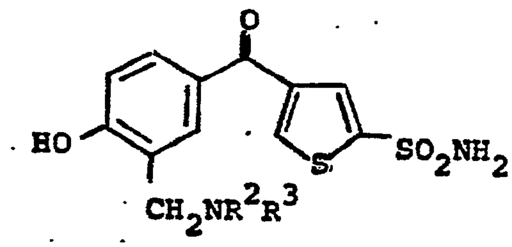

| $-NR^2R^3$ | m.p. |
|---|---|
| $-N(C_2H_5)_2 \cdot HCl$ | 95-110°C |
| $-N(CH_3)i\text{-}Bu \cdot HCl$ | 190-195°C |

EXAMPLE 7

4-(4-Methoxybenzoyl)furan-2-sulfonamide

Step A: Preparation of 3-(4-Methoxybenzoyl)furan

To 0.068 moles of N-butyllithium (in hexane) dissolved in 100 ml ether and cooled to -75°C under $N_2$ was added dropwise a solution of 10.0 g (0.068 moles) 3-bromofuran at such a rate that the temperature was <-70°C. The resulting pale yellow solution was stirred at -70°C for 1 hour and then a solution of p-methoxybenzonitrile (9.98 g, 0.075 moles) in 30 ml ether was added dropwise at <-70°C. This solution was stirred at -75°C for 1 hour, -50°C for 1 hour and then gradually allowed to warm to -20°C over 1 hour.

The reaction was quenched with 10 ml $H_2O$ followed by enough 2N HCl to dissolve the solid that formed. The aqueous solution was separated and refluxed for 2.5 hours, then it was cooled and extracted with 3 x 75 ml ether. The ether extracts were combined, washed with brine, dried and the solvent evaporated to give the title compound as an oil.

Step B: Preparation of 4-(4-Methoxybenzoyl)furan-2-sulfonic acid

To a solution of 10.1 g (0.05 mol) product from Step A in 100 ml $CH_2Cl_2$ cooled to 0-10°C was added 15.3 g (0.15 mol) acetic anhydride followed by the dropwise addition of 5.9 g (0.06 mol) concentrated $H_2SO_4$. The resulting solution was stirred at room temperature overnight.

The yellow solid was filtered off and washed with $CH_2Cl_2$ to provide the desired acid.

Step C: 4-(4-Methoxybenzoyl)furan-2-sulfonamide

To 1.86 g (0.0066 mol) or product from Step B in 20 ml tetrahydrofuran was added 0.16 g (0.0022 mol)

dimethylformamide followed by 3.17 g (0.025 mol) oxalyl chloride and this was stirred at room temperature overnight. The solvent was removed invacuo and the residue was taken up in ether, washed with $H_2O$, brine and dried. The solvent was evaporated to give the sulfonyl chloride as a yellow solid. This was taken up in 30 ml $CHCl_3$ and treated with $NH_3$ (gas) with stirring overnight. The resulting solid was collected by filtration and was purified by flash chromatography on silica gel eluting with 9/1 chloroform/methanol to provide pure product ($R_f$ 0.7) as a tan solid, m.p. 180-182°C.

Employing the procedures described in the foregoing Examples, the compounds described in Table II are prepared.

## TABLE II

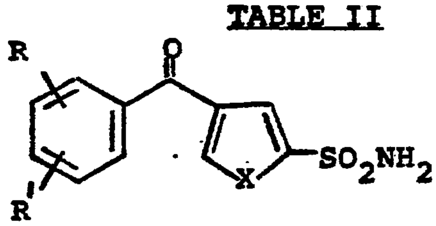

| X | R | $R^1$ |
|---|---|---|
| S | 2-$C_2H_5$ | H |
| S | 4-F | 3-$CH_2CH_2OH$ |
| S | 3-$OCH_3$ | 4-F |
| S | 2-$CH_3$ | 4-$CH_3$ |
| S | 3-$CF_3$ | H |
| O | H | 3-$CH_2CH_2OCH_3$ |
| O | H | 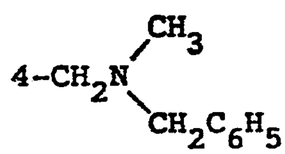 |
| O | 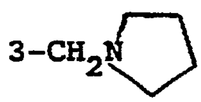 | H |
| O | H | 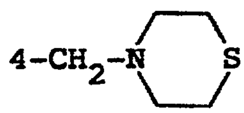 |

## EXAMPLE 8

| Solution Composition | a | b |
|---|---|---|
| Compound | 1 mg. | 15 mg. |
| Monobasic sodium phosphate .$2H_2O$ | 9.38 mg. | 6.10 mg. |
| Dibasic sodium phosphate .$12H_2O$ | 28.48 mg. | 16.80 mg. |
| Benzalkonium chloride | 0.10 mg. | 0.10 mg. |
| Water for injection q.s. ad. | 1.0 ml. | 1.0 ml. |

Compound, phosphate buffer salts, and benzalkonium chloride are added to and admixed with water. The pH of the resulting admixture is adjusted to 6.8 and the final formulation diluted to volume. The formulation is rendered sterile by appropriate means, such as starting the preparative procedure with sterile components and proceeding under sterile conditions, irradiating or autoclaving the finished formulation, or the like.

EXAMPLE 9

Compound:5 mg.
petrolatum q.s. ad.:1 gram
Compound and the petrolatum are aseptically combined.

EXAMPLE 10

Compound:1 mg.
Hydroxypropylcellulose q.s.:12 mg.
Ophthalmic inserts are manufactured from compression molded films which are prepared on a Carver Press by subjecting the powdered mixture of the above ingredients to a compressional force of 12,000 lbs. (gauge) at 300°F for one to four minutes. The film is cooled under pressure by having cold water circulate in the platen. Ophthalmic inserts are then individually cut from the film with a rod-shaped punch. Each insert is placed into a vial, which is then placed in a humidity cabinet (88% R.H. at 30°C) for two to four days. After removal from the humidity cabinet, the vials are stoppered and then capped. The vials containing the hydrate insert are then autoclaved at 250°F. for 1/2 hour.

EXAMPLE 11

- Compound0.10%
- Gelrite®0.6 %
- benzalkonium chloride0.01%
- mannitol4%
- sufficient water to make100%

**Claims**

1. A compound of structural formula:

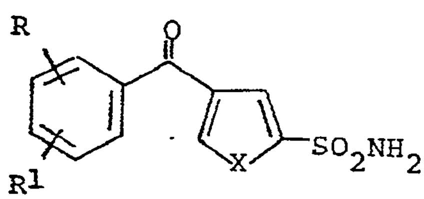

or an ophthalmologically acceptable salt thereof, wherein:
X is S or O; and
R and $R^1$ are independently
1) hydrogen;
2) hydroxy;
3) halo;
4) nitro;
5) perfluoro-$C_{1-3}$alkyl;
6) $C_{1-6}$alkyl;
7) $C_{1-6}$alkoxy;
8) $-(C_nH_{2n})-NR^2R^3$, wherein n is 0–3 and $R^2$ and $R^3$ are independently
a) hydrogen;
b) $C_{1-8}$alkyl;
c) monocyclic aryl-$C_{1-3}$alkyl;
d) hydroxy-$C_{2-6}$alkyl;
e) $C_{1-3}$alkoxy-$C_{2-6}$alkyl;
f) $R^4R^5N$-$C_{2-4}$alkyl, wherein $R^4$ and $R^5$ are independently hydrogen or $C_{1-3}$alkyl;
g) $R^2$ and $R^3$ if lower alkyl taken together either directly or through a second hetero atom selected from O, S or $NR^{20}$ form a 5- to 7- membered heterocycle with the nitrogen atom to which they are attached, $R^{20}$ representing hydrogen, $C_{1-8}$alkyl, monocyclic aryl-$C_{1-3}$alkyl, hydroxy-$C_{2-6}$alkyl, $C_{1-3}$alkoxy-$C_{2-6}$alkyl, or $R^4R^5N$-$C_{2-4}$alkyl in which $R^4$ and $R^5$ are as defined above; or

h) 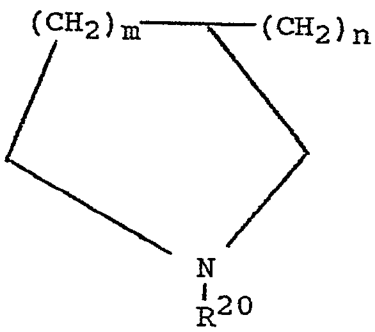

wherein m is 1 or 2, and $R^{20}$ is as defined above.

2. The compound of claim 1, wherein X is -S-.

3. The compound of claim 1, wherein one of R and $R^1$ is hydrogen or hydroxy, and the other is $-(C_nH_{2n})-NR^2R^3$, $C_{1-6}$alkyl or $C_{1-6}$alkoxy.

4. The compound of claim 2, wherein one of R and $R^1$ is hydrogen or hydroxy, and the other is $-(C_nH_{2n})-NR^2R^3$, $C_{1-6}$alkyl or $C_{1-6}$alkoxy.

5. The compound of Claim 4, wherein n is 1, and $R^2$ and $R^3$ are each $C_{1-8}$alkyl.

6. The compound of Claim 4 which is:
4-(4-methylbenzoyl)thiophenesulfonamide;
4-(4-methoxybenzoy )thiophene-2-sulfonamide;
4-(4-hydroxybenzoyl)thiop hene-2-sulfonamide;
4-(4-methylaminomethylbenzoyl)thiophene-2-sulfo amide;
4-[3-(2-pyridylmethylaminomethyl)]benzoylthi ophene-2-sulfonamide;
4-[3-(N,N-dimethylaminomethyl)-4-hydroxy]benz ylthiophene-2-sulfonamide;
4-[(4-methoxybenzoyl)fur an-2-sulfonamide; or an ophthalmologically acceptable salt thereof.

7. An ophthalmological composition for the treatment of elevated intraocular pressure and/or glaucoma comprising an ophthalmologically acceptable carrier and an effective intraocular pressure lowering and/or antiglaucoma amount of the compound of Claim 1.

8. The use of a compound of Claim 1 for the preparation of a medicament useful for treating elevated intraocular pressure and/or glaucoma.

**Patentansprüche**

1. Verbindung der Strukturformel:

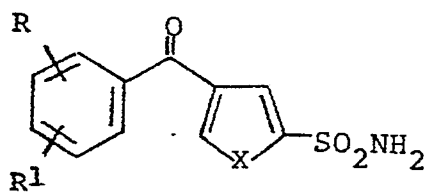

oder ein ophthalmologisch annehmbares Salz davon,
worin
X S oder O ist; und
R und $R^1$ unabhängig
1) Wasserstoff;
2) Hydroxy;
3) Halogen;
4) Nitro;
5) Perfluor-$C_{1-3}$-alkyl;
6) $C_{1-6}$-Alkyl;
7) $C_{1-6}$-Alkoxy;
8) $-(C_nH_{2n})-NR^2R^3$ sind, worin n O–3 ist und $R^2$ und $R^3$ unabhängig
a) Wasserstoff;
b) $C_{1-8}$-Alkyl;
c) monocyclisches Aryl-$C_{1-3}$-alkyl;
d) Hydroxy-$C_{2-6}$-alkyl;
e) $C_{1-3}$-Alkoxy-$C_{2-6}$-alkyl;
f) $R^4R^5N-C_{2-4}$-Alkyl, worin $R^4$ und $R^5$ unabhängig Wasserstoff oder $C_{1-3}$-Alkyl sind;

g) $R^2$ und $R^3$, falls Niederalkyl, entweder direkt oder durch ein zweites aus O, S oder $NR^{20}$ ausgewähltes Heteroatom zusammengenommen mit dem Stickstoffatom, an das sie gebunden sind, einen 5- bis 7-gliedrigen Heterocyclus bilden, wobei $R^{20}$ Wasserstoff, $C_{1-8}$-Alkyl, monocyclisches Aryl-$C_{1-3}$-Alkyl, Hydroxy-$C_{2-6}$-Alkyl, $C_{1-3}$-Alkoxy-$C_{2-6}$-Alkyl oder $R^4R^5N$-$C_{2-4}$-Alkyl, worin $R^4$ und $R^5$ wie oben definiert sind, darstellt, oder

h)

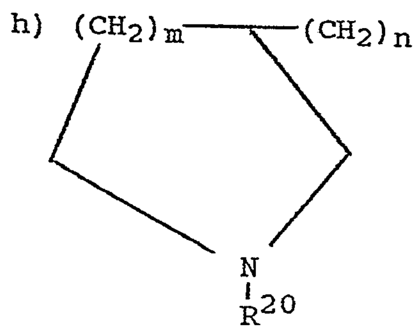

wherein m 1 oder 2 ist und $R^{20}$ wie oben definiert ist, sind.

2. Verbindung nach Anspruch 1, worin X -S- ist.

3. Verbindung nach Anspruch 1, worin eines aus R und $R^1$ Wasserstoff oder Hydroxy ist und das andere $-(C_nH_{2n})-NR^2R^3$, $C_{1-6}$-Alkyl oder $C_{1-6}$-Alkoxy ist.

4. Verbindung nach Anspruch 2, worin eines aus R und $R^1$ Wasserstoff oder Hydroxy ist und das andere $-(C_nH_{2n})-NR^2R^3$, $C_{1-6}$-Alkyl oder $C_{1-6}$-Alkoxy ist.

5. Verbindung nach Anspruch 4, worin n 1 ist und $R^2$ und $R^3$ jeweils $C_{1-8}$-Alkyl sind.

6. Verbindung nach Anspruch 4, welche
4-(4-Methylbenzoyl)thiophensulfonamid;
4-(4-Methoxybenzoyl)thiophen-2-sulfonamid;
4-(4-Hydroxybenzoyl)thiophen-2-sulfonamid;
4-(4-Methylaminomethylbenzoyl)thiophen-2-sulfonamid;
4-[3-(2-Pyridylmethylaminomethyl)]benzoylthiophen-2-sulfonamid;
4-[3-(N,N-Dimethylaminomethyl)-4-hydroxy]benzoylthiophen-2-sulfonamid;
4-(4-Methoxybenzoyl)furan-2-sulfonamid; oder ein ophthalmologisch annehmbares Salz davon ist.

7. Ophthalmologische Zusammensetzung zur Behandlung von erhöhtem Augeninnendruck und/oder Glaukom, welche einen ophthalmologisch annehmbaren Träger und eine den Augeninnendruck erniedrigende und/oder gegen Glaukom wirksame Menge der Verbindung nach Anspruch 1 umfaßt.

8. Verwendung einer Verbindung nach Anspruch 1, zur Herstellung eines bei der Behandlung von erhöhtem Augeninnendruck und/oder Glaukom geeigneten Medikaments.

**Revendications**

1. Composé de formule structurelle:

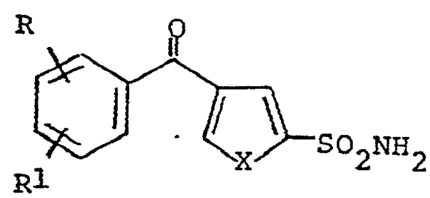

ou un sel correspondant acceptable en ophtalmologie, où:
X est S ou O; et
R et $R^1$ sont de façon indépendente
1) un hydrogène;
2) un hydroxy;
3) un halo;
4) un nitro;
5) un perfluoro-$C_{1-3}$alkyle;
6) un $C_{1-6}$alkyle;
7) un $C_{1-6}$alcoxy;
8) $-(C_nH_{2n})-NR^2R^3$, où n est 0–3 et $R^2$ et $R^3$ sont, de façon indépendante
a) un hydrogène;

b) un $C_{1-8}$alkyle;
c) un aryl-$C_{1-3}$alkyle monocyclique;
d) un hydroxy-$C_{2-6}$alkyle;
e) un $C_{1-3}$alcoxy-$C_{2-6}$alkyle;
f) un $R^4R^5N$-$C_{2-4}$alkyle; où $R^4$ et $R^5$ sont de façon indépendante l'hydrogène ou un $C_{1-3}$alkyle;
g) $R^2$ et $R^3$, s'ils représentent un alkyle inférieur, soit directement soit par un second hétéro atome choisi parmi O, S ou $NR^{20}$, forment ensemble un hétérocycle de 5 à 7 chaînons avec l'atome d'azote auquel ils sont rattachés,

$R^{20}$ représentant l'hydrogène, un $C_{1-8}$alkyle, un aryl-$C_{1-3}$alkyle monocyclique, un hydroxy-$C_{2-6}$alkyle, un $C_{1-3}$alcoxy-$C_{2-6}$alkyle, ou un $R^4R^5N$-$C_{2-4}$alkyle dans lequel $R^4$ et $R^5$ sont tels que définis ci-dessus; ou

h) 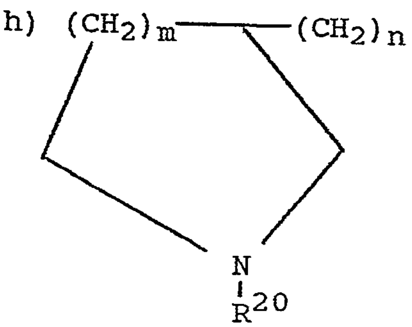

où m est 1 ou 2 et $R^{20}$ est tel que défini ci-dessus.

2. Composé selon la revendication 1, où X est -S-.

3. Composé selon la revendication 1, où un des R et $R^1$ est un hydrogène ou un hydroxy et l'autre est -($C_nH_{2n}$)-$NR^2R^3$, un $C_{1-6}$alkyle ou un $C_{1-6}$alcoxy.

4. Composé selon la revendication 2, où un des R et $R^1$ est un hydrogène ou un hydroxy, et l'autre est -($C_nH_{2n}$)-$NR^2R^3$, un $C_{1-6}$alkyle ou un $C_{1-6}$alcoxy.

5. Composé selon la revendication 4 où n est 1, et $R^2$ et $R^3$ sont chacun un $C_{1-8}$alkyle.

6. Composé selon la revendication 4 qui est:
le 4-(4-méthylbenzoyl)thiophènesulfonamide;
le 4-(4-méthoxybenzoyl)thiophène-2-sulfonamide;
le 4-(4-hydroxybenzoyl)thiophène-2-sulfonamide;
le 4-(4-méthylaminométhylbenzoyl)thiophène-2-sulfonamide;
le 4-[3-(2-pyridylméthylaminométhyl)]benzoylthiophène-2-sulfonamide;
le 4-[3-(N,N-diméthylaminométhyl)-4-hydroxy]benzoylthiophène-2-sulfonamide;
le 4-(4-méthylbenzoyl)furanne-2-sulfonamide; ou un sel correspondant acceptable en ophtalmoligie.

7. Composition ophtalmologique pour le traitement d'une hypertension intraoculaire et/ou du glaucome comportant un support acceptable en ophtalmologie et une quantité du composé de la revendication 1 telle qu'elle soit efficace pour diminuer la pression intraoculaire et/ou pour lutter contre le glaucome.

8. Emploi d'un composé de la revendication 1 pour la préparation d'un médicament utile pour traiter une hypertension intraoculaire et/ou le glaucome.